# FASCICULE DE BREVET EUROPEEN

(11) **EP 1 161 250 B1**
(45) Date de publication et mention de la délivrance du brevet: **17.08.2005**
(21) Numéro de dépôt: 00910947.1
(22) Date de dépôt: 15.03.2000
(51) Int. Cl.: A61K 35/74, A61K 39/39, A61P 37/04, A61P 35/00, C07K 14/26

(54) **FRACTIONS MEMBRANAIRES BACTERIENNES IMMUNOSTIMULANTES DANS LE TRAITEMENT DE CANCERS**
IMMUNSTIMULIERENDE BAKTERIENWANDFRAKTIONEN ZUR KREBSBEHANDLUNG
IMMUNOSTIMULANT BACTERIAL MEMBRANE FRACTIONS IN CANCER TREATMENT

(30) Priorité: 15.03.1999 FR 9903154
(43) Date de publication de la demande: 12.12.2001
(73) Titulaire: PIERRE FABRE MEDICAMENT, 92100 Boulogne-Billancourt (FR)
(72) Inventeur: LIBON, Christine, F-74160 Saint Julien en Genevois (FR); CORVAIA, Nathalie, F-74160 Saint Julien en Genevois (FR); BECK, Alain, F-74160 Collonges sous Salève (FR); BONNEFOY, Jean-Yves, F-74350 Le Sappey (FR)
(74) Mandataire: Ahner, Francis
(86) Numéro de dépôt international: PCT/FR2000/000623
(87) Numéro de publication internationale: WO 2000/054790

(56) Documents cités:
- WO-A-00/27432
- WO-A-91/12813
- FR-A- 2 471 785
- FR-A- 2 596 064
- FR-A- 2 726 472

## Description

La présente invention a pour objet l'utilisation d'une fraction membranaire de Klebsiella *pneumoniae* pour la préparation d'une composition pharmaceutique immunostimulante et capable d'induire une réponse immunitaire antitumorale, destinée au traitement et à la prévention des cancers. L'invention comprend en outre des compositions pharmaceutiques les contenant, associées à des composés anticancéreux.

La transformation d'une cellule normale en cellule maligne est le résultat de nombreux événements différents, qui peuvent se produire spontanément, comme les mutations ou les réarrangements de gènes, ou être induits par des agents chimiques, physiques ou viraux.

Les tumeurs sont infiltrées par des cellules immunocompétentes, notamment des lymphocytes, des cellules dendritiques et des macrophages.

Les macrophages associés aux tumeurs (TAM) proviennent de la circulation sanguine et sont recrutés sur le site tumoral par des cytokines. Les TAM se lient aux cellules tumorales par l'intermédiaire de glycoprotéines, sucres et phospholipides, et prolifèrent au site tumoral (J. Natl. Cancer Inst., 1998, 90:1583). Ils y sécrètent de nombreuses cytokines qui participent à leur activité antitumorale. Parmi les plus importantes, on trouve le TNF-α et l'IL-12.

L'activité antitumorale du TNF-α a été démontrée dans des modèles expérimentaux chez la souris (Beyaert R. and Fiers W., Cytokines, chapter 24, 335-360 Academic. Press. 1998) et a été testée chez l'homme pour traiter les cancers de la vessie : seul, il présente une activité modérée (Steinberg et al., Ann. Oncol., 1992, 3,741-745 ; Eur. Urol. 1992, 22:112).

La production d'IL-12 par des macrophages activés sert à moduler la réponse immunitaire en favorisant la formation de lymphocytes T CD4+ de type Th1, qui produisent de l'IL-2 et de l'IFN-γ. L'activité inhibitrice de l'IL-12 sur l'angiogenèse et la régression tumorale est bien connue, et semble liée à l'induction d'IFN-γ qui stimule la production d'IP-10 (interferon-inducible protein-10) et de MIG (monokine induced by IFN-γ) (J. Natl. Cancer Inst., 1998, 90:1583).

La thérapie à BCG (Bacille Calmette Guérin) est utilisée pour prévenir la récurrence de certains types de cancer de la vessie. Le mécanisme d'action proposé actuellement repose sur la production de cytokines : libération précoce de cytokines inflammatoires (TNF-α, IL-1, IL-6, IL-8), dans un deuxième temps production d'IL-2 et d'IFN-γ (réponse Th1), puis plus tardivement d'IL-4, d'IL-5 et d'IL-10 (réponse Th2) . Enfin, se produit une phase d'activation cellulaire avec amplification de populations cytotoxiques (Patard et al., Progrès en Urologie, 1998, 8,415-421).

Cependant, la thérapie à BCG n'a pas que des avantages, car l'efficacité parfois observée l'est au prix d'une morbidité également supérieure. De plus, il existe des contre-indications à la BCG thérapie : tuberculose active (mais pas tuberculose antérieure), immunosuppression (MV, transplantation, ...), réaction systémique antérieure au BCG (hépatite, pneumonie, BCGite), traitements aux stéroïdes. Par ailleurs, il existe des résistances ou des récurrences après une thérapie à BCG.

La fraction membranaire de K. pneumoniae I145 entre dans la composition d'une préparation pharmaceutique prévenant la survenue et la récidive d'infections respiratoires d'origine bactérienne et utilisée chez l'homme depuis 20 ans. A ce titre, il existe un recul de non toxicité du produit. L'ensemble des données citées plus haut montre qu'il existe aujourd'hui un besoin de disposer de nouveaux immunostimulants dépourvus d'activité toxique. De tels immunostimulants seraient d'un grand intérêt pour le traitement de certains types de cancer.

De manière surprenante, les auteurs de la présente invention ont mis en évidence que des fractions membranaires d'une bactérie à gram négatif, Klebsiella *pneumoniae* (dénommée FMKp), en particulier des fractions membranaires obtenues par les procédés tels que décrits ci-après dans les exemples, possèdent les propriétés immunostimulantes recherchées.

Les inventeurs ont montré de manière inattendue que la FMKp était capable non seulement de stimuler la prolifération des cellules mononucléées du sang humain, démontrant ainsi son activité immunostimulante, mais également d'induire la production de TNF-α et d'IL-12, notamment par les monocytes, cytokines impliquées dans la réponse immunitaire antitumorale.

Ainsi, la présente invention a pour objet l'utilisation d'une fraction membranaire de Klebsiella *pneumoniae,* pour la préparation d'une composition pharmaceutique immunostimulante, et capable d'induire une réponse immunitaire antitumorale, et ce quel que soit le mode d'administration in vivo choisi (voie entérale ou parentérale).

Par composé ou composition pharmaceutique immunostimulante, on entend désigner dans la présente invention un composé ou une composition pharmaceutique capable d'augmenter une réponse immune non spécifique.

Par composé ou composition pharmaceutique capable d'induire une réponse immunitaire antitumorale, on entend désigner dans la présente invention un composé ou une composition pharmaceutique capable en particulier d'accroître l'efficacité d'un composé anticancéreux ou d'accroître l'efficacité d'un traitement anticancéreux, tel que par exemple un traitement par radiothérapie.

Par fraction membranaire de Klebsiella *pneumoniae*, on entend désigner dans la présente invention toute fraction ou extrait membranaire purifié ou partiellement purifié obtenu à partir d'une culture de ladite bactérie et dont le procédé de préparation comprend au moins une étape de lyse des bactéries obtenues après culture et une étape de séparation de la fraction contenant les membranes desdites bactéries du lysat total obtenu après l'étape de lyse, notamment par centrifugation ou filtration.

Selon l'invention, les fractions membranaires pourront être préparées selon les méthodes connues de l'homme de l'art telles que par exemple la méthode décrite par Haeuw J.F. et al. (Eur. J. Biochem, 255, 446-454, 1998).

Selon un mode de réalisation particulier, l'invention est relative à une utilisation selon l'invention, caractérisée en ce que la fraction membranaire est préparée par un procédé comprenant les étapes suivantes :
a) la culture desdites bactéries dans un milieu de culture permettant leur croissance suivie d'une centrifugation de ladite culture ;
b) le cas échéant, désactivation des enzymes lytiques du culot de bactéries obtenu à l'étape a), puis centrifugation de la suspension obtenue ;
c) extraction et élimination des protéines non membranaires et des acides nucléiques du culot obtenu à l'étape a) ou b) par au moins un cycle de lavage du culot dans une solution d'extraction ;
d) digestion du culot de membranes obtenu à l'étape c) en présence d'enzymes protéolytiques suivie d'une centrifugation ;
e) au moins un cycle de lavage du culot obtenu à l'étape d) dans une solution physiologique et/ou dans de l'eau distillée ; et
f) ultrasonicadon du culot obtenu à l'étape e).

L'étape b) de désactivation des enzymes lytiques du culot de bactéries obtenu à l'étape a) peut être réalisée par toutes méthodes connues de désactivation d'enzymes, telles qu'en particulier par chauffage du culot bactérien remis en suspension à une température de préférence voisine de 100°C, ou par addition d'inhibiteur de l'activité de ces enzymes.

L'étape c) d'extraction et d'élimination des protéines non membranaires et des acides nucléiques du culot obtenu à l'étape a) ou b) peut être réalisée par exemple par au moins un cycle de lavage du culot dans une solution d'extraction correspondant à l'addition d'une solution hypertonique (solution d'extraction), de préférence une solution saline de molarité voisine de 1 M, suivie après un temps de contact suffisant pour l'effet désiré d'une centrifugation de la suspension obtenue et de l'élimination du surnageant obtenu après ladite centrifugation, ce cycle de lavage pouvant être reproduit plusieurs fois.

L'étape d) de digestion du culot de membranes obtenu à l'étape c) peut être réalisée en présence d'une solution d'enzymes protéolytiques telles que par exemple la trypsine, la chymotrypsine ou toute enzyme à activité protéolytique connue, les conditions de la réaction, pH de la solution, température et durée de la réaction, étant de préférence ajustées aux conditions optimales pour l'activité de ou des enzymes choisies, suivie d'une centrifugation, ce cycle de digestion pouvant être reproduit plusieurs fois avec la même enzyme, la même combinaison d'enzymes ou avec une enzyme différente pour chaque cycle de digestion effectué.

L'étape e) de lavage du culot obtenu à l'étape d) est réalisée par reprise du culot dans une solution physiologique ou dans de l'eau distillée suivie, après un temps de contact suffisant, d'une centrifugation, ce cycle de lavage pouvant être reproduit plusieurs fois.

Enfin, l'étape f) d'ultrasonication du culot a pour objectif en particulier de désintégrer et d'homogénéiser la fraction membranaire obtenue en fin d'étape e). Les conditions d'ultrasonication (durée et puissance) seront déterminées par l'homme de l'art en fonction par exemple de la quantité de fraction membranaire à traiter.

Selon un autre mode de réalisation particulier, l'invention est relative à une utilisation selon l'invention, caractérisée en ce que la fraction membranaire est préparée par un procédé comprenant les étapes suivantes :
a) la culture desdites bactéries dans un milieu de culture permettant leur croissance suivie le cas échéant d'une centrifugation ;
b) la congélation du milieu de culture ou du culot obtenu à l'étape a) suivie d'une décongélation et du séchage des cellules ;
c) élimination au moyen d'une DNase des acides nucléiques des cellules sèches obtenues à l'étape b) remises en suspension ;
d) broyage des cellules obtenues à l'étape c) et clarification de la suspension obtenue ;
e) précipitation en milieu acide de la suspension obtenue à l'étape d) et élimination du culot ;
f) neutralisation du surnageant obtenu à l'étape e) contenant la suspension membranaire, suivie d'une dialyse et d'une concentration de la suspension membranaire ; et
g) stérilisation de la suspension membranaire concentrée obtenue à l'étape f).

Les conditions de décongélation à l'étape b) du procédé ci-dessous seront bien entendu déterminées par l'homme de l'art en fonction de la quantité de culot initiale à traiter, de préférence réalisée à 4°C pendant au moins 48 heures pour l'équivalent de 1 Kg de cellules sèches.

A l'étape c), l'élimination des acides nucléiques est réalisée par exemple par l'addition d'une DNase, à une concentration finale de 5 mg/ml d'une suspension de cellules à une concentration équivalente à 5 % de cellules sèches.

Le broyage des cellules obtenues à l'étape c) peut être réalisé au moyen de tout système ou appareillage connu par l'homme de l'art pour le broyage de cellules tel que les presses ou de préférence tel que le broyage en boucle de Manton Gaulinet pendant 30 minutes.

La clarification de la suspension obtenue après broyage pourra être réalisée au moyen de tout système ou appareillage connu par l'homme de l'art pour la clarification de broyats cellulaires bactériens tel que le système Sharpless.

L'étape e) de précipitation en milieu acide de la suspension obtenue à l'étape d) peut être réalisée par exemple avec l'acide acétique. La précipitation est suivie par l'élimination du culot au moyen par exemple d'un système de type Sharpless et par la récupération du surnageant.

L'étape f) consiste en une étape dans laquelle le surnageant, obtenu après précipitation en milieu acide, est neutralisé, dilué, dialysé puis concentré.

Enfin, la dernière étape consiste en une étape de stérilisation du concentré de fraction membranaire obtenu à l'étape précédente comme par exemple par chauffage à 121°C pendant environ 35 minutes.

L'invention a également pour objet l'utilisation selon l'invention, caractérisée en ce que la composition pharmaceutique comprend en outre un agent permettant de véhiculer ladite fraction membranaire sous une forme permettant d'améliorer sa stabilité et/ou son activité immunostimulante et/ou sa capacité à induire une réponse immunitaire antitumorale, telle que sous la forme d'une émulsion de type huile dans eau ou eau dans huile, ou sous la forme d'une particule de type liposome, microsphère, nanosphère ou tout type de structure permettant l'encapsulation et la présentation sous forme particulaire de ladite fraction membranaire.

Est également comprise dans la présente invention, l'utilisation selon l'invention, caractérisée en ce que la composition pharmaceutique comprend en outre un agent permettant de potentialiser l'activité immunostimulante et/ou la réponse immunitaire antitumorale desdites fractions membranaires.

Parmi lesdits agents permettant de potentialiser l'activité immunostimulante et/ou la réponse immunitaire antitumorale desdites fractions membranaires, on préfère les cytokines et les composés cellulaires.

Parmi les cytokines, on peut citer, mais sans s'y limiter : l'IL-2, l'IL-12, l'IL-18, l'IFN-γ et l'IFN-α.

Parmi les composés cellulaires, on préfère notamment les acides nucléiques, les composés de la famille des ribosomes, ou encore les protéines de la famille des protéines de choc thermique.

Est également comprise dans la présente invention, l'utilisation selon l'invention, caractérisée en ce que la composition pharmaceutique comprend en outre un agent potentialisateur permettant de réguler l'activité immunostimulante et/ou la réponse immunitaire antitumorale desdites fractions membranaires.

Parmi lesdits agents potentialisateurs permettant de réguler l'activité immunostimulante et/ou la réponse immunitaire antitumorale desdites fractions membranaires, on préfère les hormones et les facteurs de croissance.

Parmi les hormones, on peut citer, mais sans s'y limiter la β-hCG.

Parmi les facteurs de croissance, on peut citer, mais sans s'y limiter : l'EGF, l'IGF-1, l'IGF-2, le GM-CSF et le G-CSF.

L'invention a également pour objet l'utilisation selon l'invention pour la préparation d'une composition pharmaceutique destinée à être administrée en association avec un traitement anticancéreux, notamment un traitement anticancéreux par chimiothérapie (mono ou polychimiothérapie) et/ou une radiothérapie.

Selon l'invention, la préparation de la composition pharmaceutique est destinée à être administrée, par voie entérale ou parentérale, simultanément, séparément ou étalée dans le temps avec le traitement anticancéreux.

L'invention comprend en outre l'utilisation selon l'invention, pour la préparation d'une composition pharmaceutique comprenant un composé à activité anticancéreuse associé à ladite fraction membranaire.

De nombreux composés à activité anticancéreuse peuvent être ainsi associés à ladite fraction membranaire immunostimulante et/ou capable d'induire une réponse immunitaire antitumorale.

Parmi ces composés, on peut citer notamment, mais sans s'y limiter, les inhibiteurs de protéases ou les composés à activité anti-angiogénique, tels que par exemple :
- les inhibiteurs de protéases tels que les TIMPs ;
ou les composés à activité anti-angiogénique suivants : l'angiostatine, l'endostatine, MCP-1, IP-10 et PF-4 ainsi que des anticorps, des antisens ou des peptides anti-VEGF, anti-angiogénine, anti-aFGF, anti-bFGF.

Ainsi, l'invention est relative à l'utilisation selon l'invention, caractérisée en ce que ledit traitement anticancéreux associé est un traitement chimiothérapeutique comprenant un inhibiteur de protéases ou un composé à activité anti-angiogénique.

L'invention a aussi pour objet l'utilisation selon l'invention, pour la préparation d'une composition pharmaceutique destinée à la prévention ou le traitement des cancers, notamment les cancers de la vessie, de la prostate, du colon, du foie, ou les mélanomes malins.

L'invention concerne en outre les compositions pharmaceutiques comprenant une fraction membranaire susceptible d'être obtenue par les procédés selon les revendications 1-3.

Le titre en protéoglycanne des fractions membranaires susceptibles d'être obtenues par lesdits procédés, principe actif de la FMKp, représenté par la somme des teneurs en galactose et en protéine, est de préférence compris :
- pour le galactose : entre 1,2 g/l et 3,4 g/l ;
- pour les protéines : entre 7,5 g/l et 14,9 g/l.

De manière plus préférée, ce titre sera :
- pour le galactose : entre 1,8 g/l et 2,6 g/l ;
- pour les protéines : entre 9,3 g/l et 11,7 g/l.

Sont également comprises dans la présente invention, les compositions pharmaceutiques comprenant une fraction membranaire de bactérie gram négatif, notamment de Klebsiella *pneumoniae,* caractérisée en ce qu'elle est associée à un traitement anticancéreux par chimiothérapie et/ou par radiothérapie.

On entend ici désigner par fraction membranaire, toute fraction membranaire de bactérie gram négatif telle que définie précédemment, dont celle susceptible d'être obtenue par les procédés selon les revendications 1-3.

De façon préférée, l'invention concerne une composition pharmaceutique selon l'invention, caractérisée en ce qu'elle contient un composé anticancéreux comme produit de combinaison pour une utilisation simultanée, séparée ou étalée dans le temps, notamment un composé anticancéreux choisi parmi les inhibiteurs de protéases ou parmi les composés présentant une activité anti-angiogénique.

De préférence, lesdites compositions pharmaceutiques selon l'invention, pourront comprendre en outre des agents tels que les véhicules, agents capables de potentialiser et/ou de réguler l'activité immunostimulante et/ou la réponse immunitaire antitumorale desdites fractions membranaires tels que définis précédemment.

Les légendes des figures et exemples qui suivent sont destinés à illustrer l'invention sans aucunement en limiter la portée.
Légendes des figures :

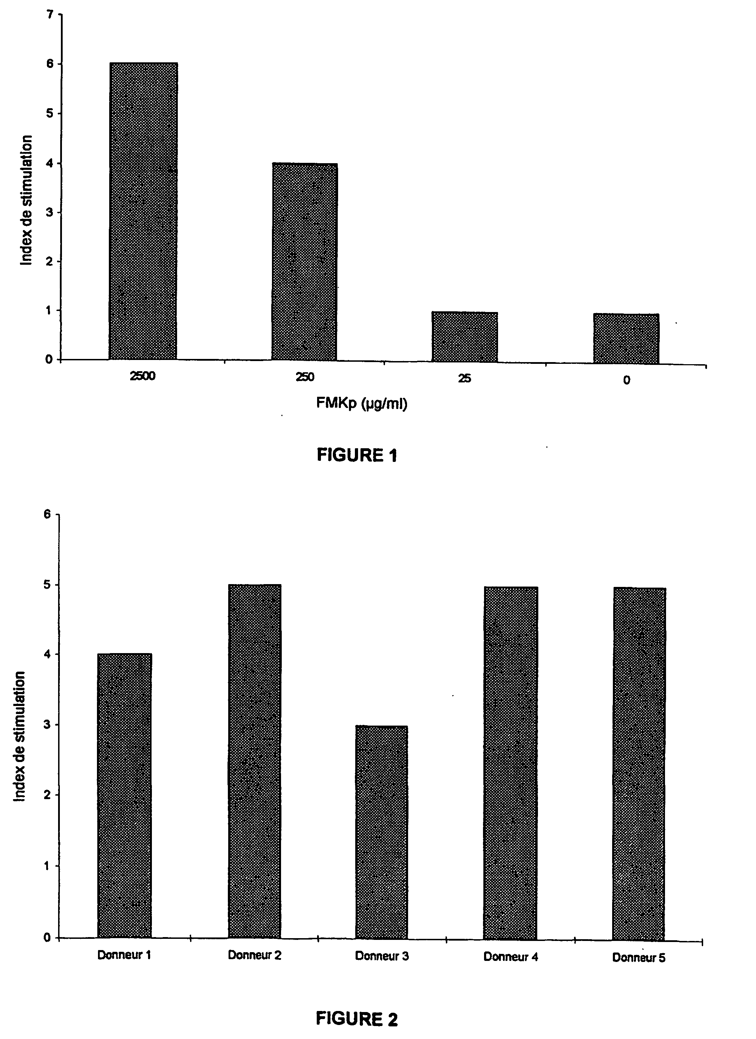

Figure 1 : Prolifération de PBMC en présence de FMKp - Etude dose-réponse
   Les cellules mononucléées (PBMC) sont obtenues par séparation à l'aide d'une solution de Ficoll-sodium métrizoate à partir du sang total. Les PBMC sont alors ensemencés à raison de 10 000 cellules/puits en présence d'agents stimulants, sous un volume total de 200 µl. Après 72 h d'incubation, la prolifération est objectivée par addition de thymidine tritiée. Les résultats sont exprimés en index de stimulation = [cpm PBMC + stimulus]/[cpm PBMC sans stimulus (= milieu RPMI + 10 % SVF)].
Figure 2 : Prolifération de PBMC en présence de FMKp - Reproductibilité de l'effet sur plusieurs donneurs (FMKp à 250 µg/ml).
Figure 3 : Production de TNF-α par des monocytes sanguins
   Les monocytes sont cultivés en milieu RPMI 1640+SVF 10 % et en présence de différentes concentrations de produit. Les cellules sont incubées dans une étuve à 37°C sous une atmosphère contenant 5 % de CO₂. Conditions de cultures : 200 000 cellules/puits, 18 h d'incubation. Après incubation, les plaques de culture sont centrifugées et les surnageants sont aliquotés et conservés à - 80°C jusqu'au dosage. Les concentrations de cytokines présentes dans les surnageants de cultures sont déterminées par ELISA (Enzyme-Linked ImmunoSorbent Assay ) : kit Predicta de Genzyme (seuil de détection à 3 pg/ml).

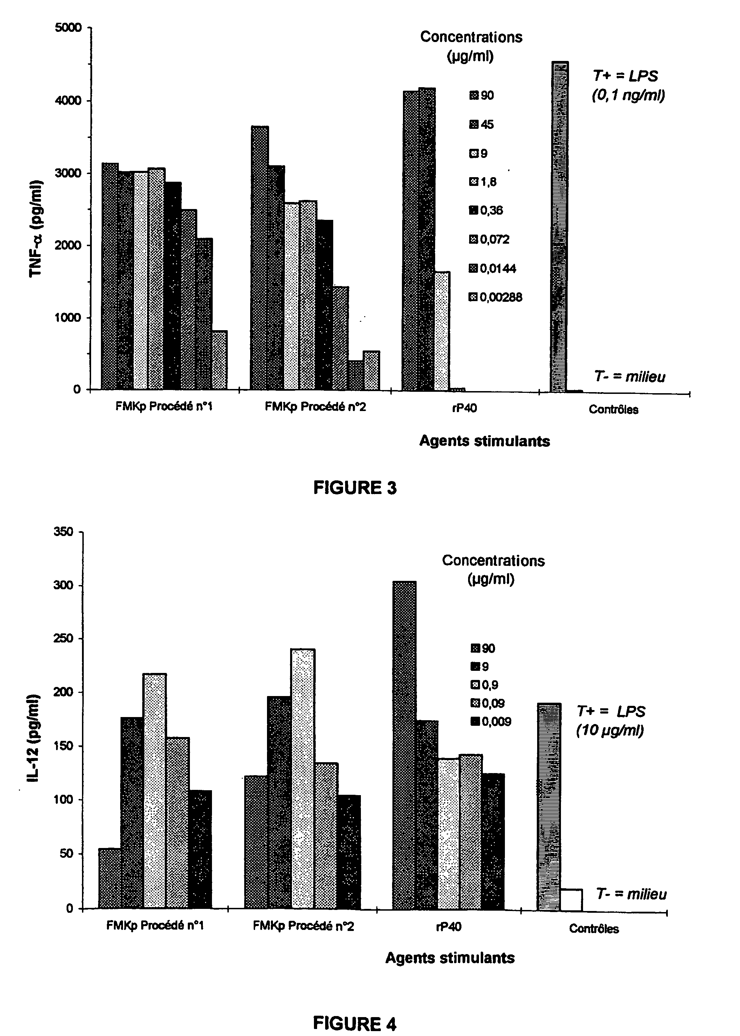

Figure 4 : Production d'IL-12 p70 (biologiquement active) par des monocytes sanguins.
   Les monocytes sont cultivés en milieu RPMI 1640+SVF 10 % et en présence de différentes concentrations de produit. Les cellules sont incubées dans une étuve à 37°C sous une atmosphère contenant 5 % de CO₂. Conditions de cultures : 500 000 cellules/puits, 24 h d'incubation. Après incubation, les plaques de culture sont centrifugées et les surnageants sont aliquotés et conservés à - 80°C jusqu'au dosage. Les concentrations de cytokines présentes dans les surnageants de cultures sont déterminées par ELISA : couple d'anticorps Endogen (seuil de détection à 15 pg/ml).

### Exemple 1 : Obtention de la faction membranaire de K. pneumoniae (FMKp)

### Procédé N° 1

L'extraction des membranes de K. pneumoniae I145 à partir du culot de centrifugation de l'étape est précédée de préférence par une étape de destruction des enzymes lytiques des composants cellulaires contenus dans le culot, par exemple par chauffage à 100°C de celui-ci, éventuellement après remise en solution.

L'extraction proprement dite des membranes à partir du culot de centrifugation est réalisée de préférence par traitement des composants cellulaires du culot, après une éventuelle destruction des enzymes lytiques, à l'aide d'une solution saline, par exemple du chlorure de sodium 1 M, une ou plusieurs fois, puis centrifugation, de préférence, à 20 000 g, de la suspension obtenue, le surnageant de cette centrifugation, qui est éliminé, contient les impuretés non membranaires telles que protéines et acides nucléiques, tandis que le culot contient les membranes.

Après séparation de la solution saline contenant les impuretés, les membranes sont digérées en présence d'enzymes protéolytiques, de préférence la trypsine et la chymotrypsine, en solution à pH 8 à 37°C pendant 4 heures.

Après digestion, la solution est homogénéisée par ultrasonication. Le produit ainsi obtenu constitue la fraction membranaire nommée FMKp.

Le surnageant obtenu est à nouveau centrifugé dans les mêmes conditions, de préférence à 140 000 g.

### Préparation des glycopeptides membranaires

Cette fraction est préparée à partir du culot obtenu par centrifugation à 40 000 g pendant 20 minutes. Ledit culot est remis en suspension dans du sérum physiologique puis cette suspension est portée pendant 10 minutes à 100°C dans un bain-marie d'eau bouillante pour désactiver les enzymes lytiques. Après refroidissement, on centrifuge 30 mn à 20 000 g. Le culot obtenu est extrait deux fois avec du NaCl 1M pour éliminer les protéines et les acides nucléiques. Les membranes sont recueillies par centrifugation durant 30 minutes à 20 000 g.

Elles sont ensuite soumises à une digestion par de la trypsine à pH 8 et à 37 °C pendant 4 heures puis par de la chymotrypsine dans les mêmes conditions.

Les membranes sont alors recueillies par centrifugation à 2 000 g pendant 30 minutes, lavées avec du sérum physiologique puis de l'eau distillée et sont soumises à une désintégration par les ultrasons de 15 minutes.

### Procédé N° 2

Après décongélation à + 4°C pendant 48 h minimum, 1 kg de cellules sèches de K. pneumoniae est remis en suspension à 5 % cellules sèches. La DNase est ajoutée à 5 mg/l On procède ensuite au broyage en boucle au Manton Gaulin pendant 30 min puis à une clarification sur SHARPLES à 50 l/h, suivie d'une précipitation à l'acide acétique à pH = 4,2 + 0,1 pendant 30 min. Le culot est éliminé (SHARPLES à 25 l/h) et le surnageant est neutralisé, dilué à 2 fois le volume initial avec de l'eau osmosée. Une dialyse à volume constant est alors effectuée sur PUF 100 jusqu'à 800 Ωcm, suivie d'une concentration de la suspension membranaire (SM) ainsi obtenue, à 11 1/kg de cellules sèches. On procède alors à l'autoclavage de la SM à + 121°C durant 35 min que l'on peut conserver à + 4°C pendant 6 semaines.

### Caractéristiques de la FMKp

Par définition, le titre en protéoglycanne, principe actif de la FMKp, est égal à la somme des teneurs en galactose et en protéines.
- Galactose : en moyenne 2,2 g /l
- Protéines : en moyenne 10,5 g/l

### Exemple 2 : Prolifération de PBMC du sang humain

Les résultats obtenus montrent que, de manière surprenante, la FMKp déclenche la prolifération de PBMC. Cet effet est dose-dépendant et maximal pour 2,5 mg/ml de FMKp (figure 1). Par ailleurs, cet effet est reproductible (figure 2).

### Exemple 3 : Production de cytokines par des monocytes purifiés du sang humain

Les monocytes humains sont obtenus à partir des cellules mononucléées (lymphocytes, monocytes, cellules NK, ...) préalablement isolées du sang total humain. L'obtention des monocytes repose sur l'expression en grande quantité de l'antigène de surface CD14 sur ces cellules. La séparation est une sélection positive. L'efficacité de la séparation magnétique des monocytes est ensuite évaluée par cytométrie en flux en effectuant un marquage avec un anticorps CD13 couplé à la fluorescéine iso-thiocyanate (FITC) : la suspension cellulaire contient alors 94 à 97 % de monocytes.

Les résultats d'études in vitro démontrent que, de manière intéressante, la FMKp est un immunostimulant qui induit la prolifération de PBMC du sang humain avec un effet direct sur les monocytes : production de TNF-α (figure 3), et d'IL-12 p70 (figure 4). Il est remarquable que la protéine P40 recombinante (rP40), l'OmpA de K. pneumoniae, est également capable de stimuler la production de TNF-α (figure 3 ), et d'IL-12 p70 (figure 4) par des monocytes humains.

## Revendications

1. Utilisation d'une fraction membranaire de Klebsiella pneumoniae comprenant des protéoglycannes pour la préparation d'une composition pharmaceutique immunostimulante et capable d'induire une réponse immunitaire antitumorale pour la prévention et le traitement des cancers, **caractérisée en ce que** ladite fraction membranaire est obtenue par un procédé comprenant une étape de lyse des bactéries K. pneumoniae après leur culture et une étape de séparation de la fraction contenant les membranes desdites bactéries du lysat total obtenu après l'étape de lyse.

2. Utilisation selon la revendication 1, **caractérisée en ce que** la fraction membranaire est préparée par un procédé comprenant les étapes suivantes :
a) la culture desdites bactéries dans un milieu de culture permettant leur croissance suivie d'une centrifugation de ladite culture ;
b) le cas échéant, désactivation des enzymes lytiques du culot de bactéries obtenu à l'étape a), puis centrifugation de la suspension obtenue ;
c) extraction et élimination des protéines non membranaires et des acides nucléiques du culot obtenu à l'étape a) ou b) par au moins un cycle de lavage du culot dans une solution d'extraction ;
d) digestion du culot de membranes obtenu à l'étape c) en présence d'enzymes protéasiques suivie d'une centrifugation ;
e) au moins un cycle de lavage du culot obtenu à l'étape d) dans une solution physiologique et/ou dans de l'eau distillée ; et
f) ultrasonication du culot obtenu à l'étape e).

3. Utilisation selon la revendication 1, **caractérisée en ce que** la fraction membranaire est préparée par un procédé comprenant les étapes suivantes ;
a) la culture desdites bactéries dans un milieu de culture permettant leur croissance suivie le cas échéant d'une centrifugation ;
b) la congélation du milieu de culture ou du culot obtenu à l'étape a) suivie d'une décongélation et du séchage des cellules ;
c) élimination au moyen d'une DNase des acides nucléiques des cellules sèches obtenues à l'étape b) remises en suspension ;
d) broyage des cellules obtenues à l'étape c) et clarification de la suspension obtenue ;
e) précipitation en milieu acide de la suspension obtemie à l'étape d) et élimination du culot ;
f) neutralisation du surnageant obtenu à l'étape e) contenant la suspension membranaire, suivie d'une dialyse et d'une concentration de la suspension membranaire ; et
g) stérilisation de la suspension membranaire concentrée obtenue à l'étape f).

4. Utilisation selon l'une des revendications 1 à 3, **caractérisée en ce que** la composition pharmaceutique comprend en outre un agent permettant de véhiculer ladite fraction membranaire sous une forme permettant d'améliorer sa stabilité et/ou son activité immunostimulante et/ou sa capacité d'induire une réponse immunitaire antitumorale.

5. Utilisation selon la revendication 4, **caractérisée en ce que** ledit agent est de type émulsion huile dans eau ou eau dans huile.

6. Utilisation selon la revendication 5, **caractérisée en ce que** ledit agent est sous la forme d'une particule de type liposome, microsphère, nanosphère ou tout type de structure permettant l'encapsulation et la présentation sous forme particulaire de ladite fraction membranaire.

7. Utilisation selon l'une des revendications 1 à 6, **caractérisée en ce que** la composition pharmaceutique comprend en outre un agent permettant de potentialiser l'activité immunostimulante et/ou la réponse immunitaire antitumorale desdites fractions membranaires.

8. Utilisation selon la revendication 7, **caractérisée en ce que** l'agent permettant de potentialiser l'activité immunostimulante et/ou la réponse immunitaire antitumorale desdites fractions membranaires est une cytokiue.

9. Utilisation selon la revendication 7, **caractérisée en ce que** l'agent permettant de potentialiser l'activité immunostimulante et/ou la réponse immunitaire antitumorale desdites fractions membranaires est un agent de régulation choisi parmi les hormones.

10. Utilisation selon la revendication 7, **caractérisée en ce que** l'agent permettant de potentialiser l'activité immunostimulante et/ou la réponse immunitaire antitumorale desdites fractions membranaires est un agent de régulation choisi parmi les facteurs de croissance.

11. Utilisation selon la revendication 7, **caractérisée en ce que** l'agent permettant de potentialiser l'activité immunostimulante et/ou la réponse immunitaire antitumorale desdites fractions membranaires est un composé cellulaire.

12. Utilisation selon la revendication 11, **caractérisée en ce que** ledit composé cellulaire est un acide nucléique choisi parmi les ADN et les ARN.

13. Utilisation selon la revendication 11, **caractérisée en ce que** ledit composé cellulaire est un composé de la famille des ribosomes.

14. Utilisation selon la revendication 11, **caractérisée en ce que** ledit composé cellulaire est une protéine de la famille des protéines de choc thermique.

15. Utilisation selon l'une des revendications 1 à 14, pour la préparation d'une composition pharmaceutique destinée à être administrée en association avec un traitement anticancéreux.

16. Utilisation selon la revendication 15, **caractérisée en ce que** le traitement anticancéreux est une chimiothérapie et/ou une radiothérapie.

17. Utilisation selon l'une des revendications 15 et 16, pour la préparation d'une composition pharmaceutique destinée à être administrée simultanément, séparément ou étalée dans le temps avec le traitement anticancéreux.

18. Utilisation selon la revendication 17, **caractérisée en ce que** la composition pharmaceutique est destinée à être administrée par voie entérale ou parentérale.

19. Utilisation selon l'une des revendications 15 à 18, **caractérisée en ce que** ledit traitement anticancéreux associé est un traitement chimiothérapeutique comprenant un inhibiteur de protéases ou un composé à activité anti-angiogénique.

20. Utilisation selon l'une des revendications 1 à 19, pour la prévention et/ou le traitement des cancers de la vessie, de la prostate, du colon, du foie et des mélanomes malins.

21. composition pharmaceutique comprenant une fraction menabranaire de Klebsiella pneumoniae comprenant des protéoglycannes obtenus par un procédé de préparation d'une fraction membranaire tel que décrit dans les revendications 1 à 3, **caractérisée en ce qu'**elle contient un composé anticancéreux comme produit de combinaison pour une utilisation simultanée, séparée ou étalée dans le temps.

22. Composition pharmaceutique selon la revendication 21, **caractérisée en ce que** ledit composé anticancéreux est choisi parmi les inhibiteurs de protéases ou parmi les composés présentant une activité anti-angiogénique.

## Claims

1. Use of a membrane fraction of *Klebsiella pneumoniae* comprising proteoglycans for preparing a pharmaceutical composition which is immunostimulant and capable of inducing an antitumor immune response for the prevention and treatment of cancers, **characterized in that** said membrane fraction is obtained using a method comprising a step of lysis of the *K.pneumoniae* bacteria after having cultured them, and a step of separation of the fraction containing the membranes of said bacteria from the total lysate obtained after the lysis step.

2. Use according to Claim 1, **characterized in that** the membrane fraction is prepared using a method comprising the following steps:
a) culturing of said bacteria in a culture medium which allows their growth, followed by centrifugation of said culture;
b) where appropriate, deactivation of the lytic enzymes of the bacterial pellet obtained in step a), then centrifugation of the suspension obtained;
c) extraction and elimination of the non-membrane-bound proteins and of the nucleic acids of the pellet obtained in step a) or b) with at least one cycle of washing the pellet in an extraction solution;
d) digestion of the membrane pellet obtained in step c) in the presence of protease enzymes, followed by centrifugation;
e) at least one cycle of washing the pellet obtained in step d) in a physiological solution and/or in distilled water; and
f) ultrasonication of the pellet obtained in step e).

3. Use according to Claim 1, **characterized in that** the membrane fraction is prepared using a method comprising the following steps:
a) culturing of said bacteria in a culture medium which allows their growth, followed, where appropriate, by centrifugation;
b) freezing of the culture medium or of the pellet obtained in step a), followed by thawing and drying of the cells;
c) elimination, using a DNase, of the nucleic acids from the dried cells obtained in step b), which have been resuspended;
d) grinding of the cells obtained in step c) and clarification of the suspension obtained;
e) precipitation, in acid medium, of the suspension obtained in step d) and elimination of the pellet;
f) neutralization of the supernatant obtained in step e) containing the membrane suspension, followed by dialysis and concentration of the membrane suspension; and
g) sterilization of the concentrated membrane suspension obtained in step f).

4. Use according to one of Claims 1 to 3, **characterized in that** the pharmaceutical composition also comprises an agent for vehiculing said membrane fraction in a form which makes it possible to improve its stability and/or its immunostimulant activity and/or its capacity to induce an antitumor immune response.

5. Use according to Claim 4, **characterized in that** said agent is of the oil-in-water or water-in-oil emulsion type.

6. Use according to Claim 5, **characterized in that** said agent is in the form of a particle of the liposome, microsphere or nanosphere type, or any type of structure which enables said membrane fraction to be encapsulated and presented in particulate form.

7. Use according to one of Claims 1 to 6, **characterized in that** the pharmaceutical composition also comprises an agent for potentiating the immunostimulant activity and/or the antitumor immune response of said membrane fractions.

8. Use according to Claim 7, **characterized in that** the agent for potentiating the immunostimulant activity and/or the antitumor immune response of said membrane fractions is a cytokine.

9. Use according to Claim 7, **characterized in that** the agent for potentiating the immunostimulant activity and/or the antitumor immune response of said membrane fractions is a regulatory agent chosen from hormones.

10. Use according to Claim 7, **characterized in that** the agent for potentiating the immunostimulant activity and/or the antitumor immune response of said membrane fractions is a regulatory agent chosen from growth factors.

11. Use according to Claim 7, **characterized in that** the agent for potentiating the immunostimulant activity and/or the antitumor immune response of said membrane fractions is a cellular compound.

12. Use according to Claim 11, **characterized in that** said cellular compound is a nucleic acid chosen from DNAs and RNAs.

13. Use according to Claim 11, **characterized in that** said cellular compound is a compound of the ribosome family.

14. Use according to Claim 11, **characterized in that** said cellular compound is a protein of the heat-shock protein family.

15. Use according to one of Claims 1 to 14, for preparing a pharmaceutical composition intended to be administered in combination with an anticancer treatment.

16. Use according to Claim 15, **characterized in that** the anticancer treatment is chemotherapy and/or radiotherapy.

17. Use according to either of Claims 15 and 16, for preparing a pharmaceutical composition intended to be administered simultaneously with, separately from or spread out over time with the anticancer treatment.

18. Use according to Claim 17, **characterized in that** the pharmaceutical composition is intended to be administered via the enteral or parenteral route.

19. Use according to one of Claims 15 to 18, **characterized in that** said combined anticancer treatment is a chemotherapeutic treatment comprising a protease inhibitor or a compound with anti-angiogenic activity.

20. Use according to one of Claims 1 to 19, for preventing and/or treating bladder cancers, prostate cancers, colon cancers, liver cancers and malignant melanomas.

21. Pharmaceutical composition comprising a membrane fraction of *Klebsiella pneumoniae* comprising proteoglycans obtained using a method for preparing a membrane fraction as described in Claims 1 to 3, **characterized in that** it contains an anticancer compound as a combination product for use which is simultaneous, separate or spread out over time.

22. Pharmaceutical composition according to Claim 21, **characterized in that** said anticancer compound is chosen from protease inhibitors or from compounds with anti-angiogenic activity.

## Patentansprüche

1. Verwendung einer Proteoglykane umfassenden Membranfraktion von Klebsiella pneumoniae für die Herstellung einer pharmazeutischen Zusammensetzung, welche immunstimulierend wirkt und in der Lage ist, eine gegen einen Tumor gerichtete Immunantwort zu induzieren, für die Verhütung und die Behandlung von Krebserkrankungen, **dadurch gekennzeichnet, dass** die Membranfraktion erhalten wird durch ein Verfahren, welches einen Schritt einer Lyse der K. pneumoniae-Bakterien nach deren Kultur und einen Schritt einer Abtrennung der Fraktion, welche die Membranen der Bakterien enthält, von dem nach dem Lyseschritt erhaltenen Gesamtlysat umfasst.

2. Verwendung nach Anspruch 1, **dadurch gekennzeichnet, dass** die Membranfraktion hergestellt wird durch ein Verfahren, welches die folgenden Schritte umfasst:
a) die Kultur der Bakterien in einem Kulturmedium, welches deren Vermehrung erlaubt, gefolgt von einer Zentrifugation der Kultur;
b) gegebenenfalls eine Inaktivierung der lytischen Enzyme des Bodensatzes von Bakterien, welcher in Schritt a) erhalten wird, dann Zentrifugation der erhaltenen Suspension;
c) Extraktion und Entfernung der Nicht-Membran-Proteine und der Nukleinsäuren aus dem in Schritt a) oder b) erhaltenen Bodensatz durch wenigstens einen Waschzyklus des Bodensatzes in einer Extraktionslösung;
d) Verdau des in Schritt c) erhaltenen Bodensatzes von Membranen in Gegenwart von Proteaseenzymen, gefolgt von einer Zentrifugation;
e) wenigstens einen Waschzyklus des in Schritt d) erhaltenen Bodensatzes in einer physiologischen Lösung und/oder in destilliertem Wasser; und
f) Ultraschallbehandlung des in Schritt e) erhaltenen Bodensatzes.

3. Verwendung nach Anspruch 1, **dadurch gekennzeichnet, dass** die Membranfraktion hergestellt wird durch ein Verfahren, welches die folgende Schritte umfasst:
a) die Kultur der Bakterien in einem Kulturmedium, welches deren Vermehrung erlaubt, gegebenenfalls gefolgt von einer Zentrifugation;
b) das Einfrieren des Kulturmediums oder des Bodensatzes, das bzw. der in Schritt a) erhalten wird, gefolgt von einem Auftauen und der Trocknung der Zellen;
c) Entfernung der Nukleinsäuren von den in Schritt b) erhaltenen getrockneten Zellen, die resuspendiert worden sind, mittels einer DNase;
d) Aufbrechen der in Schritt c) erhaltenen Zellen und Klärung der erhaltenen Suspension;
e) Ausfällung der in Schritt d) erhaltenen Suspension in saurem Milieu und Entfernung des Bodensatzes;
f) Neutralisation des in Schritt e) erhaltenen Überstands, welcher die Membransuspension enthält, gefolgt von einer Dialyse und einer Aufkonzentrierung der Membransuspension; und
g) Sterilisation der in Schritt f) erhaltenen konzentrierten Membransuspension.

4. Verwendung nach einem der Ansprüche 1 bis 3, **dadurch gekennzeichnet, dass** die pharmazeutische Zusammensetzung außerdem ein Mittel umfasst, welches erlaubt, die Membranfraktion in einer Form vorzulegen, die erlaubt, deren Stabilität und/oder deren immunstimulierende Aktivität und/oder deren Fähigkeit, eine gegen einen Tumor gerichtete Immunantwort zu induzieren, zu verbessern.

5. Verwendung nach Anspruch 4, **dadurch gekennzeichnet, dass** das Mittel vom Typ Öl-in-Wasser- oder Wasser-in-Öl-Emulsion ist.

6. Verwendung nach Anspruch 5, **dadurch gekennzeichnet, dass** das Mittel in Form eines Teilchens vom Typ Liposom, Mikrosphäre, Nanosphäre oder einer jeglichen Art von Struktur, welche die Verkapselung und die Präsentation der Membranfraktion in partikulärer Form erlaubt, vorliegt.

7. Verwendung nach einem der Ansprüche 1 bis 6, **dadurch gekennzeichnet, dass** die pharmazeutische Zusammensetzung außerdem ein Mittel umfasst, welches erlaubt, die immunstimulierende Aktivität und/oder die gegen einen Tumor gerichtete Immunantwort der Membranfraktionen zu verstärken.

8. Verwendung nach Anspruch 7, **dadurch gekennzeichnet, dass** das Mittel, welches erlaubt, die immunstimulierende Aktivität und/oder die gegen einen Tumor gerichtete Immunantwort der Membranfraktionen zu verstärken, ein Zytokin ist.

9. Verwendung nach Anspruch 7, **dadurch gekennzeichnet, dass** das Mittel, welches erlaubt, die immunstimulierende Aktivität und/oder die gegen einen Tumor gerichtete Immunantwort der Membranfraktionen zu verstärken, ein Regulationsmittel, welches unter den Hormonen ausgewählt wird, ist.

10. Verwendung nach Anspruch 7, **dadurch gekennzeichnet, dass** das Mittel, welches erlaubt, die immunstimulierende Aktivität und/oder die gegen einen Tumor gerichtete Immunantwort der Membranfraktionen zu verstärken, ein Regulationsmittel, welches unter den Wachstumsfaktoren ausgewählt wird, ist.

11. Verwendung nach Anspruch 7, **dadurch gekennzeichnet, dass** das Mittel, welches erlaubt, die immunstimulierende Aktivität und/oder die gegen einen Tumor gerichtete Immunantwort der Membranfraktionen zu verstärken, eine zelluläre Verbindung ist.

12. Verwendung nach Anspruch 11, **dadurch gekennzeichnet, dass** die zelluläre Verbindung eine aus den DNA und den RNA ausgewählte Nukleinsäure ist.

13. Verwendung nach Anspruch 11, **dadurch gekennzeichnet, dass** die zelluläre Verbindung eine Verbindung aus der Familie der Ribosomen ist.

14. Verwendung nach Anspruch 11, **dadurch gekennzeichnet, dass** die zelluläre Verbindung ein Protein aus der Familie der Hitzeschockproteine ist.

15. Verwendung nach einem der Ansprüche 1 bis 14 für die Herstellung einer pharmazeutischen Zusammensetzung, welche dafür bestimmt ist, in Verbindung mit einer Antikrebs-Behandlung verabreicht zu werden.

16. Verwendung nach Anspruch 15, **dadurch gekennzeichnet, dass** die Antikrebs-Behandlung eine Chemotherapie und/oder eine Strahlentherapie ist.

17. Verwendung nach einem der Ansprüche 15 und 16 für die Herstellung einer pharmazeutischen Zusammensetzung, welche dafür bestimmt ist, gleichzeitig, getrennt oder zeitlich gestaffelt mit der Antikrebs-Behandlung verabreicht zu werden.

18. Verwendung nach Anspruch 17, **dadurch gekennzeichnet, dass** die pharmazeutische Zusammensetzung dazu bestimmt ist, auf enteralem oder parenteralem Wege verabreicht zu werden.

19. Verwendung nach einem der Ansprüche 15 bis 18, **dadurch gekennzeichnet, dass** die damit kombinierte Antikrebs-Behandlung eine chemotherapeutische Behandlung ist, welche einen Inhibitor von Proteasen oder eine Verbindung mit anti-angiogenetischer Aktivität umfasst.

20. Verwendung nach einem der Ansprüche 1 bis 19 für die Verhütung und/oder die Behandlung von Krebserkrankungen der Blase, der Prostata, des Kolons, der Leber oder von malignen Melanomen.

21. Pharmazeutische Zusammensetzung, umfassend eine Membranfraktion von Klebsiella pneumoniae, welche Proteoglykane umfasst, welche erhalten werden durch ein Verfahren zur Herstellung einer Membranfraktion, wie in den Ansprüchen 1 bis 3 beschrieben, **dadurch gekennzeichnet, dass** sie eine Antikrebs-Verbindung enthält, als Kombinationsprodukt für eine gleichzeitige, getrennte oder zeitlich gestaffelte Verwendung

22. Pharmazeutische Zusammensetzung nach Anspruch 21, **dadurch gekennzeichnet, dass** die Antikrebs-Verbindung unter den Inhibitoren von Proteasen oder unter den Verbindungen, die eine antiangiogenetische Aktivität aufweisen, ausgewählt wird.


### LISTAGE DE SEQUENCES

<110> PIERRE FABRE MEDICAMENT
<120> UTILISATION DE FRACTIONS MEMBRANAIRES BACTERIENNES A ACTIVITE IMMUNOSTIMULANTE DANS LE TRAITEMENT DE CANCERS, LEURS PROCEDES DE PREPARATION ET LES COMPOSITIONS PHARMACEUTIQUES LES CONTENANT.
<130> D17974
<140> FR 99 03154
<141> 1999-03-15
<160> 4
<170> PatentIn Vers. 2.0
<210> 1
<211> 1035
<212> ADN
<213> Klebsiella pneumoniae
<220>
<221> exon
<222> (1)..(1032)
<220>
<221> intron
<222> (1033)..(1035)
<220>
<221> CDS
<222> (1)..(1032)
<400> 1
   atg aaa gca att ttc gta ctg aat gcg gct ccg aaa gat aac acc tgg     48
   Met Lys Ala Ile Phe Val Leu Asn Ala Ala Pro Lys Asp Asn Thr Trp
    1               5                  10                  15
   tat gca ggt ggt aaa ctg ggt tgg tcc cag tat cac gac acc ggt ttc     96
   Tyr Ala Gly Gly Lys Leu Gly Trp Ser Gln Tyr His Asp Thr Gly Phe
               20                  25                  30
   tac ggt aac ggt ttc cag aac aac aac ggt ccg acc cgt aac gat cag    144
   Tyr Gly Asn Gly Phe Gln Asn Asn Asn Gly Pro Thr Arg Asn Asp Gln
           35                  40                  45
   ctt ggt gct ggt gcg ttc ggt ggt tac cag gtt aac ccg tac ctc ggt    192
   Leu Gly Ala Gly Ala Phe Gly Gly Tyr Gln Val Asn Pro Tyr Leu Gly
       50                  55                  60
   ttc gaa atg ggt tat gac tgg ctg ggc cgt atg gca tat aaa ggc agc    240
   Phe Glu Met Gly Tyr Asp Trp Leu Gly Arg Met Ala Tyr Lys Gly Ser
   65 70 75 80
   gtt gac aac ggt gct ttc aaa gct cag ggc gtt cag ctg acc gct aaa 288
   Val Asp Asn Gly Ala Phe Lys Ala Gln Gly Val Gln Leu Thr Ala Lys
   85 90 95
   ctg ggt tac ccg atc act gac gat ctg gac atc tac acc cgt ctg ggc 336
   Leu Gly Tyr Pro Ile Thr Asp Asp Leu Asp Ile Tyr Thr Arg Leu Gly
   100 105 110
   ggc atg gtt tgg cgc gct gac tcc aaa ggc aac tac gct tct acc ggc 384
   Gly Met Val Trp Arg Ala Asp Ser Lys Gly Asn Tyr Ala Ser Thr Gly
   115 120 125
   gtt tcc cgt agc gaa cac gac act ggc gtt tcc cca gta ttt gct ggc 432
   Val Ser Arg Ser Glu His Asp Thr Gly Val Ser Pro Val Phe Ala Gly
   130 135 140
   ggc gta gag tgg gct gtt act cgt gac atc gct acc cgt ctg gaa tac 480
   Gly Val Glu Trp Ala Val Thr Arg Asp Ile Ala Thr Arg Leu Glu Tyr
   145 150 155 160
   cag tgg gtt aac aac atc ggc gac gcg ggc act gtg ggt acc cgt cct 528
   Gln Trp Val Asn Asn Ile Gly Asp Ala Gly Thr Val Gly Thr Arg Pro
   165 170 175
   gat aac ggc atg ctg agc ctg ggc gtt tcc tac cgc ttc ggt cag gaa 576
   Asp Asn Gly Met Leu Ser Leu Gly Val Ser Tyr Arg Phe Gly Gln Glu
   180 185 190
   gat gct gca ccg gtt gtt gct ccg gct ccg gct ccg gct ccg gaa gtg 624
   Asp Ala Ala Pro Val Val Ala Pro Ala Pro Ala Pro Ala Pro Glu Val
   195 200 205
   gct acc aag cac ttc acc ctg aag tct gac gtt ctg ttc aac ttc aac 672
   Ala Thr Lys His Phe Thr Leu Lys Ser Asp Val Leu Phe Asn Phe Asn
   210 215 220
   aaa gct acc ctg aaa ccg gaa ggt cag cag gct ctg gat cag ctg tac 720
   Lys Ala Thr Leu Lys Pro Glu Gly Gln Gln Ala Leu Asp Gln Leu Tyr
   225 230 235 240
   act cag ctg agc aac atg gat ccg aaa gac ggt tcc gct gtt gtt ctg 768
   Thr Gln Leu Ser Asn Met Asp Pro Lys Asp Gly Ser Ala Val Val Leu
   245 250 255
   ggc tac acc gac cgc atc ggt tcc gaa gct tac aac cag cag ctg tct 816
   Gly Tyr Thr Asp Arg Ile Gly Ser Glu Ala Tyr Asn Gln Gln Leu Ser
   260 265 270
   gag aaa cgt gct cag tcc gtt gtt gac tac ctg gtt gct aaa ggc atc 864
   Glu Lys Arg Ala Gln Ser Val Val Asp Tyr Leu Val Ala Lys Gly Ile
   275 280 285
   ccg gct ggc aaa atc tcc gct cgc ggc atg ggt gaa tcc aac ccg gtt 912
   Pro Ala Gly Lys Ile Ser Ala Arg Gly Met Gly Glu Ser Asn Pro Val
   290 295 300
   act ggc aac acc tgt gac aac gtg aaa gct cgc gct gcc ctg atc gat 960
   Thr Gly Asn Thr Cys Asp Asn Val Lys Ala Arg Ala Ala Leu Ile Asp
   305 310 315 320
   tgc ctg gct ccg gat cgt cgt gta gag atc gaa gtt aaa ggc tac aaa 1008
   Cys Leu Ala Pro Asp Arg Arg Val Glu Ile Glu Val Lys Gly Tyr Lys
   325 330 335
   gaa gtt gta act cag ccg gcg ggt taa 1035
   Glu Val Val Thr Gln Pro Ala Gly
   340
<210> 2
   <211> 344
   <212> PRT
   <213> Klebsiella pneumoniae
<400> 2
   Met Lys Ala Ile Phe Val Leu Asn Ala Ala Pro Lys Asp Asn Thr Trp
   1 5 10 15
   Tyr Ala Gly Gly Lys Leu Gly Trp Ser Gln Tyr His Asp Thr Gly Phe
   20 25 30
   Tyr Gly Asn Gly Phe Gln Asn Asn Asn Gly Pro Thr Arg Asn Asp Gln
   35 40 45
   Leu Gly Ala Gly Ala Phe Gly Gly Tyr Gln Val Asn Pro Tyr Leu Gly
   50 55 60
   Phe Glu Met Gly Tyr Asp Trp Leu Gly Arg Met Ala Tyr Lys Gly Ser
   65 70 75 80
   Val Asp Asn Gly Ala Phe Lys Ala Gln Gly Val Gln Leu Thr Ala Lys
   85 90 95
   Leu Gly Tyr Pro Ile Thr Asp Asp Leu Asp Ile Tyr Thr Arg Leu Gly
   100 105 110
   Gly Met Val Trp Arg Ala Asp Ser Lys Gly Asn Tyr Ala Ser Thr Gly
   115 120 125
   Val Ser Arg Ser Glu His Asp Thr Gly Val Ser Pro Val Phe Ala Gly
   130 135 140
   Gly Val Glu Trp Ala Val Thr Arg Asp Ile Ala Thr Arg Leu Glu Tyr
   145 150 155 160
   Gln Trp Val Asn Asn Ile Gly Asp Ala Gly Thr Val Gly Thr Arg Pro
   165 170 175
   Asp Asn Gly Met Leu Ser Leu Gly Val Ser Tyr Arg Phe Gly Gln Glu
   180 185 190
   Asp Ala Ala Pro Val Val Ala Pro Ala Pro Ala Pro Ala Pro Glu Val
   195 200 205
   Ala Thr Lys His Phe Thr Leu Lys Ser Asp Val Leu Phe Asn Phe Asn
   210 215 220
   Lys Ala Thr Leu Lys Pro Glu Gly Gln Gln Ala Leu Asp Gln Leu Tyr
   225 230 235 240
   Thr Gln Leu Ser Asn Met Asp Pro Lys Asp Gly Ser Ala Val Val Leu
   245 250 255
   Gly Tyr Thr Asp Arg Ile Gly Ser Glu Ala Tyr Asn Gln Gln Leu Ser
   260 265 270
   Glu Lys Arg Ala Gln Ser Val Val Asp Tyr Leu Val Ala Lys Gly Ile
   275 280 285
   Pro Ala Gly Lys Ile Ser Ala Arg Gly Met Gly Glu Ser Asn Pro Val
   290 295 300
   Thr Gly Asn Thr Cys Asp Asn Val Lys Ala Arg Ala Ala Leu Ile Asp
   305 310 315 320
   Cys Leu Ala Pro Asp Arg Arg Val Glu Ile Glu Val Lys Gly Tyr Lys
   325 330 335
   Glu Val Val Thr Gln Pro Ala Gly
   340